(19) Europäisches Patentamt
European Patent Office
Office européen des brevets

(11) Veröffentlichungsnummer: **0 164 699**
**A2**

(12)

# EUROPÄISCHE PATENTANMELDUNG

(21) Anmeldenummer: **85107029.2**

(22) Anmeldetag: **07.06.85**

(51) Int. Cl.⁴: **C 07 J 53/00**
A 61 K 31/565

(30) Priorität: **12.06.84 DE 3422187**

(43) Veröffentlichungstag der Anmeldung:
**18.12.85 Patentblatt 85/51**

(84) Benannte Vertragsstaaten:
**AT BE CH DE FR GB IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65(DE)**

(72) Erfinder: **Wiechert, Rudolf, Prof. Dr.**
**Petzower Strasse 8 A**
**D-1000 Berlin 39(DE)**

(72) Erfinder: **Kerb, Ulrich, Dr.**
**Prinzregentenstrasse 7**
**D-1000 Berlin 31(DE)**

(72) Erfinder: **Nishino, Yukishige, Dr.**
**Lanzendorfer Weg 14**
**D-1000 Berlin 22(DE)**

(54) **1,2Beta-Methylen-4-androsten-und 4,6-androstadien-3,17-dione.**

(57) 1,2β-Methylen-4-androsten- und 4,6-androstadien-3,17-dione der allgemeinen Formel I

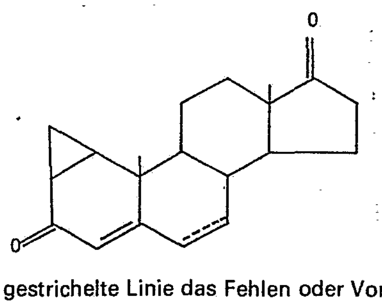

(I)

worin die gestrichelte Linie das Fehlen oder Vorhandensein einer zusätzlichen Doppelbindung in 6,7-Stellung bedeutet.

Die neuen Verbindungen der allgemeinen Formel I sind als Inhibitoren der Östrogenbiosynthese zur Fertilitätskontrolle und zur Behandlung von Krankheiten geeignet, die durch Östrogene hervorgerufen werden.

Die Erfindung betrifft 1,2ß-Methylen-4-androsten- und 4,6-androstadien-3,17-dione der allgemeinen Formel I, Verfahren zu deren Herstellung und diese enthaltende pharmazeutische Präparate.

Es wurde gefunden, daß die neuen Verbindungen der allgemeinen Formel I die Östrogenbiosynthese hemmen. Das ist überraschend; denn die entsprechenden in 1,2ß-Stellung nicht methylenierten Verbindungen werden zu starken Östrogenen aromatisiert (Klinische und Experimentelle Urologie Band 3, W. Zuckschwerdt-Verlag München, 1981, Seite 126).

Bei der Biosynthese von Östrogenen spielt die Umwandlung von Androgenen zu Östrogenen eine wichtige Rolle. Diese Umwandlung verläuft über eine Reihe von Reaktionen, die zusammengefaßt als "Aromatisierung" bezeichnet werden. Das Enzym Aromatase ist das geschwindigkeitsbestimmende Enzym bei der Umwandlung von Androstendion und Testosteron zu Östron und Östradiol.

Die folgenden Verbindungen wurden auf Aromatasehemmwirkung untersucht:

A: 4-Hydroxy-4-androsten-3,17-dion, Endocrinology 100 (1977) 1684 - 1695 (als Vergleichssubstanz).

B: 1,2ß-Methylen-4-androsten-3,17-dion.

C: 1,2ß-Methylen-androsta-4,6-dien-3,17-dion.

Die durch PMSG (Pregnant Mare's Serum Gonadotropin) vermehrte Sekretion von Östradiol kann durch Applikation von Aromatasehemmern vermindert werden.

Versuch und Auswertung wurden wie folgt durchgeführt:

20 Tage alte weibliche Ratten wurden alle 2 Tage insgesamt 7 mal mit 100 I.U. PMSG subcutan vorbehandelt. Eine Stunde vor und 8 Stunden nach der letzten PMSG-Applikation ($d_{12}$) erhielten die Tiere die Testsubstanz durch Injektion. 24 Stunden nach der letzten PMSG-Applikation wurden die Tiere getötet. Im Serum wurde dann Östradiol radioimmunologisch bestimmt. In der folgenden Tabelle wird die Östradiolkonzentration in nMol/l und die Standardabweichung angegeben.

T a b e l l e

| Substanz | Dosis mg/Tier 2 x s.c. | Östradiol-konzentration im Serum nMol/l | % Hemmung (rel.Wirksamkeit) |
|---|---|---|---|
| PMSG-Kontrolle | - | 6,16 ± 3,22 | - |
| A | 3 | 1,87 ± 0,87 *) | 70 |
| B | 3 | 0,96 ± 0,45 *) | 84 |
| C | 3 | 1,16 ± 1,12 *) | 81 |

*) = signifikanter Unterschied gegen die PMSG-Kontrolle

Aus der Tabelle ist ersichtlich, daß die erfindungsgemäßen Substanzen B und C einen stärkeren Abfall der Östradiolkonzentration bewirken als die bekannte Substanz A.

Als Aromataseinhibitoren sind die neuen Verbindungen der allgemeinen Formel I zur Hemmung der Östrogenbiosynthese und damit zur Behandlung von Krankheiten geeignet, die durch Östrogene bedingt oder von Östrogenen abhängig sind.

Einen Östrogenüberschuß findet man häufig bei Frauen in der Menopause mit einem Risiko, an Brustkrebs zu erkranken.

Bei Männern führt eine vermehrte Östrogenproduktion und ein erhöhtes Östrogen/Androgen-Verhältnis zur Gynäkomastie und zur Prostatahyperplasie.

Die Verbindungen eignen sich somit zur Behandlung von Brustkrebs und anderer östrogeninduzierter oder -stimulierter Tumoren.

Aromataseinhibitoren sind auch vorteilhaft geeignet zur prophylaktischen und/oder therapeutischen Behandlung der Prostatahyperplasie.

Wahrscheinlich sind Aromatasehemmer auch zur Behandlung des drohenden Herzinfarkts geeignet, da erhöhte Östrogenspiegel beim Mann einem Herzinfarkt vorangehen.

Die neuen Verbindungen sind auch wertvoll zur Beeinflussung der Fertilität, beispielsweise zur Behandlung der männlichen Infertilität, die aus erhöhten Östrogenspiegeln resultiert. Ferner können die Verbindungen als Antifertilitätsmittel verwendet werden, um Ovulation oder Implantation bei Frauen im fortpflanzungsfähigen Alter zu verhindern.

Die Verbindungen können oral oder parenteral, beispielsweise intraperitoneal, intramuskulär, subkutan oder perkutan, verabreicht werden. Die Verbindungen können auch in das Gewebe implantiert werden.

Die zu verabreichende Menge der Verbindung schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 - 100 mg/kg Körpergewicht, vorzugsweise 0,1 - 20 mg/kg Körpergewicht, je Tag betragen.

Zur oralen Verabreichung kommen Kapseln, Pillen, Tabletten, Dragées usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten. Die einzelnen Dosierungseinheiten für die orale Applikation können beispielsweise 10 bis 100 mg des Wirkstoffs (Aromataseinhibitors) enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind zum Beispiel Olivenöl, Erdnußöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Die neuen Verbindungen der allgemeinen Formel I werden erfindungsgemäß nach dem Verfahren gemäß Anspruch 5 hergestellt.

Die Verseifung der 17ß-Alkanoyloxygruppe erfolgt in üblicher Weise, beispielsweise mit einer anorganischen Base in alkoholischer Lösung unter Erhitzen am Rückfluß. Als anorganische Basen sind Natrium- und Kaliumcarbonat besonders geeignet.

Die Oxydation der 17ß-Hydroxygruppe wird in an sich bekannter Weise, zum Beispiel mit Chromsäurereagenzien (Jones'-Reagenz oder Chromsäure-Pyridin) durchgeführt.

Die Einführung der 6,7-Doppelbindung gelingt durch Allylbromierung und anschließende Bromwasserstoffabspaltung.

Die Allylbromierung wird zum Beispiel mit N-Bromsuccinimid, N-Bromacetamid, 1,3-Dibrom-5,5-dimethylhydantoin oder Dibromtetrachlorethan in Gegenwart eines Radikalbildners wie Dibenzoylperoxid in einem Lösungsmittel vorgenommen. Als Lösungsmittel kommen vorzugsweise chlorierte Kohlenwasserstoffe, wie zum Beispiel Tetrachlorkohlenstoff, Chloroform oder Tetrachlorethylen infrage. Die Umsetzung erfolgt bei erhöhter Temperatur, vorzugsweise bei der Siedetemperatur der Lösung.

Die Bromwasserstoffabspaltung unter Ausbildung der $\Delta^6$-Doppelbindung erfolgt durch Erhitzen der 6-Bromverbindung mit basischen Mitteln, vorzugsweise mit Lithiumbromid und Lithiumcarbonat oder mit Lithiumbromid und Calciumcarbonat in einem aprotischen Lösungsmittel wie Dimethylformamid bei Temperaturen von 50 bis 120 °C. Eine weitere Möglichkeit der HBr-Abspaltung besteht darin, daß man die 6-Bromverbindung in Collidin oder Lutidin erhitzt.

Falls in 17-Stellung des Ausgangssteroids der allgemeinen Formel II eine Alkanoyloxygruppe vorliegt, kann diese in üblicher Weise verseift und die freie 17-Hydroxygruppe in an sich bekannter Weise oxydiert werden.

Die Einführung der Doppelbindungen in 4,5- und 6,7-Stellung kann nach bekannten Methoden mit Dehydrierungsmitteln, wie Dichlordicyanobenzochinon, vorgenommen werden. Zweckmäßigerweise arbeitet man mit einem Überschuß an Dichlordicyanobenzochinon in einem inerten hochsiedenden Lösungsmittel wie Toluol oder Dioxan in der Siedehitze.

Beispiel 1

1,2ß-Methylen-4-androsten-3,17-dion

a) 5 g 17ß-Acetoxy-1,2ß-methylen-4-androsten-3-on (hergestellt wie in Chem. Ber. 99, 1118, (1966) beschrieben) werden in 55 ml Methanol mit einer Lösung von 5,5 g Kaliumcarbonat in 13,8 ml Wasser versetzt und 90 Minuten unter Argon am Rückfluß erhitzt. Es wird mit 5,5 ml Eisessig neutralisiert, in Eiswasser gefällt, das Produkt abgesaugt und getrocknet. Man erhält so 4,35 g 17ß-Hydroxy-1,2ß-methylen-4-androsten-3-on vom Schmelzpunkt 242 - 244 °C.

b) 3,31 g 17ß-Hydroxy-1,2ß-Methylen-4-androsten-3-on werden in 24 ml Pyridin mit 9,43 g Chromsäure-Pyridin Komplex 5 Stunden bei Raumtemperatur gerührt. Anschließend wird das Pyridin im Vakuum abdestilliert. Der Rückstand wird mit Methylenchlorid aufgeschlämmt, über Aluminiumoxid filtriert, mit Methylenchlorid nachgewaschen und das Filtrat eingedampft. Nach Umkristallisation aus Aceton-Hexan erhält man 3,1 g 1,2ß-Methylen-4-androsten-3,17-dion vom Schmelzpunkt 215 - 216,5 °C.

Beispiel 2

1,2ß-Methylen-androsta-4,6-dien-3,17-dion

500 mg 1,2ß-Methylen-4-androsten-3,17-dion werden in 30 ml Tetrachlorkohlenstoff gelöst, mit 358 mg N-Bromsuccinimid und 20 mg Dibenzoylperoxid versetzt und 45 Minuten unter Rückfluß erhitzt. Nach Abkühlung auf 30 °C wird das ausgefallene Succinimid abfiltriert, das Filtrat mit Wasser gewaschen und im Vakuum eingedampft.

Das Rohprodukt (780 mg) wird in 10 ml Dimethylformamid gelöst, 500 mg Lithiumbromid und 425 mg Lithiumcarbonat werden zugegeben und 3,5 Stunden bei 90 °C Badtemperatur gerührt. Anschließend werden die anorganischen Salze angesaugt, mit Aceton gewaschen und das Filtrat im Vakuum eingeengt. Nach Chromatographie an Silicagel, Gradientenelution mit Toluol-Aceton erhält man 210 mg 1,2ß-Methylen-androsta-4,6-dien-3,17-dion, das nach Umkristallisation aus Ether-Pentan bei 163,5 - 166 °C schmilzt.

Beispiel 3

1,2ß-Methylen-androsta-4,6-dien-3,17-dion

a) 1,16 g 17ß-Acetoxy-1,2ß-methylen-4-androsten-3-on werden in 50 ml Tetrachlorkohlenstoff gelöst, mit 2 g Calciumcarbonat, 1,22 g N-Bromsuccinimid und 40 mg Dibenzoylperoxid versetzt und 3 Stunden unter Rückfluß erhitzt. Nach dem Abkühlen auf Raumtemperatur werden das ausgefallene Succinimid und die Calciumsalze abgesaugt, das Filtrat wird mit Wasser gewaschen und im Vakuum eingedampft. Nach Chromatographie an Silicagel erhält man 920 mg 6ß-Brom-17ß-acetoxy-1,2ß-methylen-4-androsten-3-on.

b) 800 mg 6ß-Brom-17ß-acetoxy-1,2ß-methylen-4-androsten-3-on werden in 10 ml Dimethylformamid mit 1 g Lithiumcarbonat 4 Stunden bei 120 °C Badtemperatur unter Argon erhitzt. Nach dem Aufarbeiten, wie in Beispiel 2 beschrieben, erhält man 560 mg 17ß-Acetoxy-1,2ß-methylen-androsta-4,6-dien-3-on.

c) 450 mg 17ß-Acetoxy-1,2ß-methylen-androsta-4,6-dien-3-on werden verseift und oxydiert nach Beispiel 1. Man erhält so 380 mg 1,2ß-Methylen-androsta-4,6-dien-3,17-dion, die nach Umkristallisation aus Aceton-Hexan bei 165 - 166,5 °C schmelzen.

## Beispiel 4

### 1,2ß-Methylen-androsta-4,6-dien-3,17-dion

530 mg 17ß-Acetoxy-1,2ß-methylen-5ß-androstan-3-on werden in 10 ml Toluol mit 1,1 g Dichlordicyanobenzochinon 20 Stunden unter Rückfluß erhitzt. Nach dem Abkühlen wird mit Methylenchlorid verdünnt, mit Natriumcarbonat-Lösung und Wasser gewaschen, getrocknet und eingeengt. Nach Chromatographie an Silicagel erhält man 270 mg amorphes 17ß-Acetoxy-1,2ß-methylen-androsta-4,6-dien-3-on.

Nach Verseifen und Oxydieren, wie in Beispiel 1 beschrieben, erhält man 1,2ß-Methylen-androsta-4,6-dien-3,17-dion.

0164699

Patentansprüche für die Vertragsstaaten
BE, CH, LI, DE, FR, GB, IT, LU, NL, SE

1.) 1,2ß-Methylen-4-androsten- und 4,6-androstadien-3,17-dione der allgemeinen Formel I

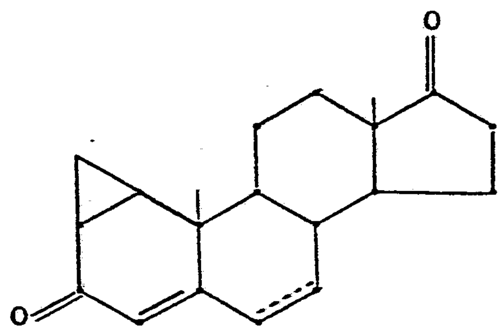

(I),

worin die gestrichelte Linie das Fehlen oder Vorhandensein einer zusätzlichen Doppelbindung in 6,7-Stellung bedeutet.

2.) 1,2ß-Methylen-4-androsten-3,17-dion.

3.) 1,2ß-Methylen-androsta-4,6-dien-3,17-dion.

4.) Pharmazeutische Präparate, gekennzeichnet durch den Gehalt an einer Verbindung gemäß Anspruch 1 bis 3.

5.) Verfahren zur Herstellung von 1,2ß-Methylen-4-androsten- und 4,6-androstadien-3,17-dionen der allgemeinen Formel I

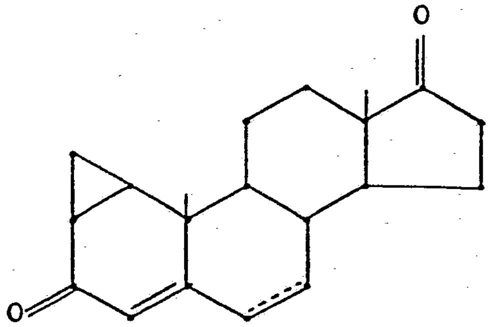

(I),

worin die gestrichelte Linie das Fehlen oder Vorhandensein einer zusätzlichen Doppelbindung in 6,7-Stellung bedeutet, dadurch gekennzeichnet, daß man in einem 17-Acyloxysteroid der allgemeinen Formel II

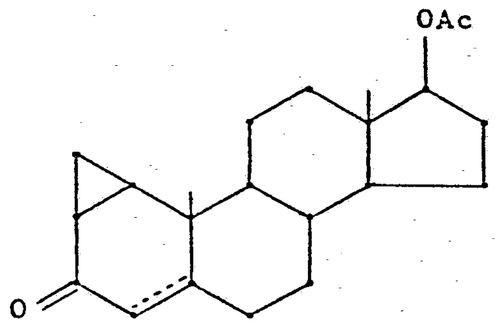

(II),

worin Ac eine $C_1$ - $C_4$-Alkanoylgruppe darstellt,

a) bei Vorliegen einer Doppelbindung in 4,5-Stellung die 17-Alkanoyloxygruppe verseift und die freigesetzte 17-Hydroxygruppe oxydiert und gegebenenfalls vor oder nach Verseifung und Oxydation die 6,7-Doppelbindung einführt oder

b) bei Vorliegen der gesättigten 5ß-H-Verbindung Doppelbindungen in 4,5- und 6,7-Stellung einführt, anschließend die 17-Alkanoyloxygruppe verseift und die freigesetzte 17-Hydroxygruppe oxydiert.

Patentanspruch für den Vertragsstaat AT

Verfahren zur Herstellung von 1,2ß-Methylen-4-androsten- und 4,6-androstadien-3,17-dionen der allgemeinen Formel I

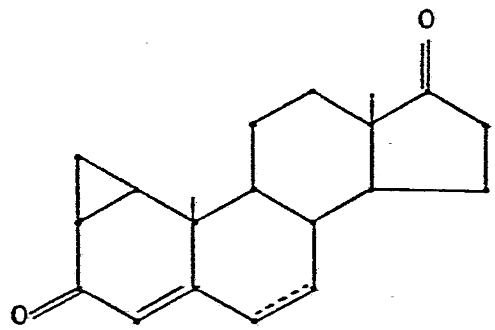

(I),

worin die gestrichelte Linie das Fehlen oder Vorhandensein einer zusätzlichen Doppelbindung in 6,7-Stellung bedeutet, dadurch gekennzeichnet, daß man in einem 17-Acyloxysteroid der allgemeinen Formel II

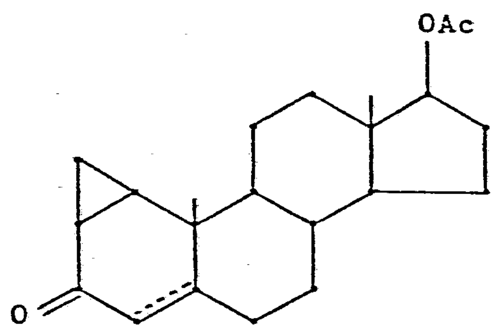

(II),

worin Ac eine $C_1$ - $C_4$-Alkanoylgruppe darstellt,

a) bei Vorliegen einer Doppelbindung in 4,5-Stellung die 17-Alkanoyloxygruppe verseift und die freigesetzte 17-Hydroxygruppe oxydiert und gegebenenfalls vor oder nach Verseifung und Oxydation die 6,7-Doppelbindung einführt oder

b) bei Vorliegen der gesättigten 5ß-H-Verbindung Doppelbindungen in 4,5- und 6,7-Stellung einführt, anschließend die 17-Alkanoyloxygruppe verseift und die freigesetzte 17-Hydroxygruppe oxydiert.